# EUROPEAN PATENT APPLICATION

(11) **EP 4 560 002 A1**
(43) Date of publication of application: **28.05.2025**
(21) Application number: 23842894.0
(22) Date of filing: 12.07.2023
(51) Int. Cl.: C12M 1/00, C12M 1/34, C12N 1/00, C12N 5/07, C12Q 1/06

(54) **CELL CULTURE DEVICE AND METHOD FOR CALCULATING CELL NUMBER**

(30) Priority: 21.07.2022 JP 2022116656; 16.06.2023 JP 2023099514
(71) Applicant: Sinfonia Technology Co., Ltd., Tokyo 105-8564 (JP)
(72) Inventor: IMATAKA, Hirohito, Tokyo 105-8564 (JP); YAMAUCHI, Takuya, Tokyo 105-8564 (JP); SUDA, Yoshimasa, Tokyo 105-8564 (JP); URABE, Yuji, Tokyo 105-8564 (JP); KANO, Shingo, Tokyo 105-8564 (JP)
(74) Representative: Angerhausen, Christoph
(86) International application number: PCT/JP2023/025791
(87) International publication number: WO 2024/018971

(57) **Abstract**

The purpose of the present disclosure is to accurately calculate the number of cells. A cell culture device 1 includes a sensor 26 for measuring the lactic acid concentration in a medium. The sensor measures the concentration of lactic acid in a medium in a culture vessel 21 during the cultivation of cells as a first physical quantity. The sensor also measures the output value of the lactic acid concentration in the medium in a supply bag 23, i.e., the output value of the lactic acid concentration in the medium supplied to the culture vessel 21, as an error output value. Further, the cell culture device 1 includes a calculator 14 that calculates the number of cells in the culture vessel 21 on the basis of the value of the corrected physical quantity that is calculated by subtracting the error output value from the value of the first physical quantity.

## Description

### TECHNICAL FIELD

The present disclosure relates to a cell culture device and a method of calculating a number of cells.

### BACKGROUND

Conventionally, a cell culture device for culturing cells in a culture vessel is known. For example, Patent Document 1 discloses a cell culture device having a culture vessel (the second vessel in Patent Document 1) in which cells and a liquid medium for cell culture are accommodated.

In the cell culture device as disclosed in Patent Document 1, a number of cells in the culture vessel is calculated appropriately during cell culture, and the culture is terminated when the number of cells reaches a target cell number. Examples of a method of calculating a number of cells may include a method of predicting a number of cells based on a lactic acid concentration in a culture medium in a culture vessel, which is measured by a sensor. Lactic acid is a substance discharged by cell metabolism, and a discharge amount of lactic acid increases as a number of cells increases. Since the number of cells corresponding to the discharge amount of lactic acid is fixed according to a type of the cells and a composition of the culture medium, the number of cells can be predicted by measuring the lactic acid concentration in the culture medium.

As a sensor for measuring a lactic acid concentration, an enzyme sensor utilizing an enzyme reaction as disclosed in Patent Document 2 (the biosensor in Patent Document 2) is generally used. The enzyme sensor in Patent Document 2 includes an oxidoreductase that reacts with lactic acid, which is a substance to be measured and is one of metabolites of cells, and an electron transfer substance that transfers electrons obtained by oxidation-reduction reaction of the lactic acid to a measurement electrode. The enzyme sensor can quantify a lactic acid concentration in a culture medium by measuring electrons, which are generated by reaction between the lactic acid in the culture medium and the oxidoreductase and are transferred to the measurement electrode.

### [Prior Art Documents]

### [Patent Documents]

Patent Document 1: Japanese Patent Laid-open Publication No. 2021-087364
Patent Document 2: Japanese Patent Laid-open Publication No. 2019-002738

However, when the lactic acid concentration in the culture medium in the culture vessel is measured by the enzyme sensor, a measurement value may be larger than a lactic acid concentration based on lactic acid actually discharged from the cells. The reason is because, separately from the lactic acid discharged by the metabolism of the cells, interfering substances, which are other substances originally contained in the culture medium, react with the enzyme sensor unintentionally. As a result, the enzyme sensor errorneously recognizes, in addition to an output value derived from the lactic acid contained in the culture medium, an output value derived from the interfering substances as the lactic acid concentration, thereby generating an error in a measurement value of the lactic acid concentration. When the error is generated in the measurement value of the lactic acid concentration as described above, an error is also generated in the number of cells predicted based on the lactic acid concentration. Thus, the number of cells in the culture vessel cannot be calculated accurately.

In addition, the above-described problem is not limited to the case where the number of cells is calculated by measuring the lactic acid concentration, but may also occur when a method of calculating a number of cells by measuring physical quantities relating to metabolites of cells is adopted.

### SUMMARY

The present disclosure provides some embodiments of a cell culture device and a method of calculating a number of cells, which are capable of accurately calculating the number of cells.

A cell culture device according to the present disclosure includes: a culture vessel configured to accommodate cells and a culture medium to culture the cells; a sensor configured to measure, with respect to a physical quantity of a metabolite of the cells that increases with proliferation of the cells, a first physical quantity, which is the physical quantity in the culture medium in the culture vessel during the culture of the cells, and an error output value, which is an output value of the physical quantity in the culture medium supplied to the culture vessel; and a cell number calculator configured to calculate a number of cells in the culture vessel based on a corrected physical quantity calculated by subtracting the error output value from a value of the first physical quantity.

According to the present disclosure, the corrected physical quantity is calculated by subtracting the error output value derived from interfering substances originally contained in the culture medium from the value of the first physical quantity in the culture medium in the culture vessel. Thus, it is possible to obtain the corrected physical quantity as a physical quantity of a metabolite of cells, which is not affected by the interfering substances contained in the culture medium and is associated with the number of cells in the culture vessel. Therefore, by deriving the number of cells based on the corrected physical quantity, the number of cells in the culture vessel can be calculated accurately.

In the cell culture device according to the present disclosure, the sensor may measure both the first physical quantity and the error output value.

Even sensors of the same type may be different in measurement capability due to difference in lots, and the like. Thus, when the lactic acid concentration in the culture medium in the culture vessel and the lactic acid concentration in the culture medium supplied to the culture vessel are measured by different sensors, measurement errors may be generated between the sensors. According to the present embodiment, since the lactic acid concentration in the culture medium in the culture vessel and the lactic acid concentration in the culture medium supplied to the culture vessel are measured by the single sensor, it is possible to eliminate measurement errors between sensors.

In the cell culture device according to the present disclosure, the culture medium in the culture vessel may be replaced during the culture, and the sensor may be measure, as the error output value, an output value of the physical quantity in the culture medium newly supplied to the culture vessel when the replacement of the culture medium is performed.

The sensor measuring the physical quantity may deteriorate as the measurement is repeated and the measurement capability may be reduced. In such a case, a deviation is generated in the measurement value of the physical quantity according to timings of the measurement by the sensor. When a timing of measuring the first physical quantity by the sensor and a timing of measuring the error output value by the sensor are significantly different, the first physical quantity and the error output value are measured by the sensor having variation in measurement capability. The corrected physical quantity calculated from the first physical quantity and the error output value, which are measured by the sensor having variation in measurement capability as described above, may deviate from an actual value. According to the present disclosure, the sensor re-measures, as the error output value, the output value of the physical quantity in the culture medium newly supplied to the culture vessel at the timing of replacing the culture medium. Therefore, it is possible to suppress a significant deviation between the timing of measuring the first physical quantity in the culture medium in the culture vessel during the culture, and the timing of measuring the error output value. Thus, it is possible to suppress a deviation of the corrected physical quantity from the actual value caused by variation in measurement capability of the sensor. By deriving the number of cells based on the corrected physical quantity obtained as described above, the number of cells in the culture vessel can be calculated accurately.

In the cell culture device according to the present disclosure, the physical quantity may be a lactic acid concentration.

In general, a concentration of a substance with a large total amount can be measured more accurately than a concentration of a substance with a small total amount. In the present disclosure, the lactic acid concentration is measured as the physical quantity. Since a discharge amount of lactic acid is as a metabolite of cells is large, the lactic acid concentration can be measured more accurately than concentrations of other substances.

A cell number calculation method according to the present disclosure is a method of calculating the number of cells in a culture vessel configured to accommodate the cells and a culture medium and culture the cells, the method including: a first measurement process of measuring, with respect to a physical quantity of a metabolite of the cells that increases with proliferation of the cells, a first physical quantity which is the physical quantity in the culture medium in the culture vessel during the culture of the cells; a second measurement process of measuring an error output value which is an output value of the physical quantity in the culture medium supplied to the culture vessel; and a cell number calculation process of calculating the number of cells in the culture vessel based on a corrected physical quantity calculated by subtracting the error output value from a value of the first physical quantity.

According to the present disclosure, the corrected physical quantity is calculated by subtracting the error output value derived from interfering substances originally contained in the culture medium from the value of the first physical quantity in the culture medium in the culture vessel. Thus, it is possible to obtain the corrected physical quantity as a physical quantity of a metabolite of cells, which is not affected by the interfering substances contained in the culture medium and is associated with the number of cells in the culture vessel. Therefore, by deriving the number of cells based on the corrected physical quantity, the number of cells in the culture vessel can be calculated accurately.

In the cell number calculation method according to the present disclosure, the culture medium in the culture vessel may be replaced during the culture, and in the second measurement process, an output value of the physical quantity in the culture medium newly supplied to the culture vessel when the replacement of the culture medium may be measured as the error output value.

The sensor measuring the physical quantity may deteriorate as the measurement is repeated and the measurement capability may be reduced. In such a case, a deviation is generated in the measurement value of the physical quantity according to timings of the measurement by the sensor. When a timing of measuring the first physical quantity by the sensor and a timing of measuring the error output value by the sensor are significantly different, the first physical quantity and the error output value are measured by the sensor having variation in measurement capability. The corrected physical quantity calculated from the first physical quantity and the error output value, which are measured by the sensor having variation in measurement capability as described above, may deviate from an actual value. According to the present disclosure, the sensor re-measures, as the error output value, the output value of the physical quantity in the culture medium newly supplied to the culture vessel at the timing of replacing the culture medium. Therefore, it is possible to suppress a significant deviation between the timing of measuring the first physical quantity in the culture medium in the culture vessel during the culture, and the timing of measuring the error output value. Thus, it is possible to suppress a deviation of the corrected physical quantity from the actual value caused by variation in measurement capability of the sensor. By deriving the number of cells based on the corrected physical quantity obtained as described above, the number of cells in the culture vessel can be calculated accurately.

### BRIEF DESCRIPTION OF DRAWINGS

FIG. 1 is a schematic front view showing a configuration of a cell culture device according to the present embodiment.
FIG. 2 is a schematic view showing individual components disposed in a cultivator.
FIG. 3 is a view showing a state of the cultivator when a cell suspension is sent from a cell bag to a culture vessel.
FIG. 4 is a view showing a state of the cultivator when a lactic acid concentration in a culture medium in a supply bag is measured.
FIG. 5 is a view showing a state of the cultivator when the culture medium is sent from the supply bag to the culture vessel.
FIG. 6 is a view showing a state of the cultivator when a lactic acid concentration in the culture medium in the culture vessel is measured.
FIG. 7 is a view showing a state of the cultivator when the culture medium is sent from the culture vessel to a drainage bag.
FIG. 8 is a view showing a state of the cultivator when the cell suspension is sent from the culture vessel to a recovery bag.
FIG. 9 is a flowchart of culturing cells in the cell culture device.
FIG. 10 is a flowchart of culturing cells in a cell culture device of a first modification.

### DETAILED DESCRIPTION

Hereinafter, embodiments of the present disclosure will now be described with reference to the accompanying drawings.

### (Cell Culture Device 1)

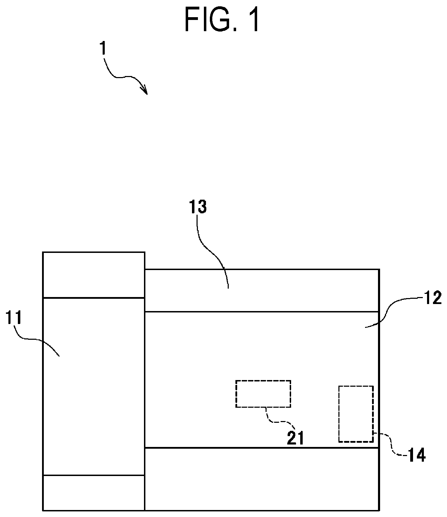

A cell culture device 1 is a device that automatically cultures cells while adjusting a culture environment. As shown in FIG. 1, the cell culture device 1 includes a refrigerating storage 11 configured to store reagents such as a culture medium and a stripping solution, a cultivator 12 provided with a culture vessel 21 disposed therein and configured to perform cell culture while adjusting an internal environment, a controller 13 configured to execute control so that culture and sub-culture of the cells, replacement of the culture medium in the culture vessel 21, recovery of a cell suspension, and the like are performed in the cultivator 12, and a calculator 14 (cell number calculation means in the present disclosure) configured to calculate a number of cells in the culture vessel 21.

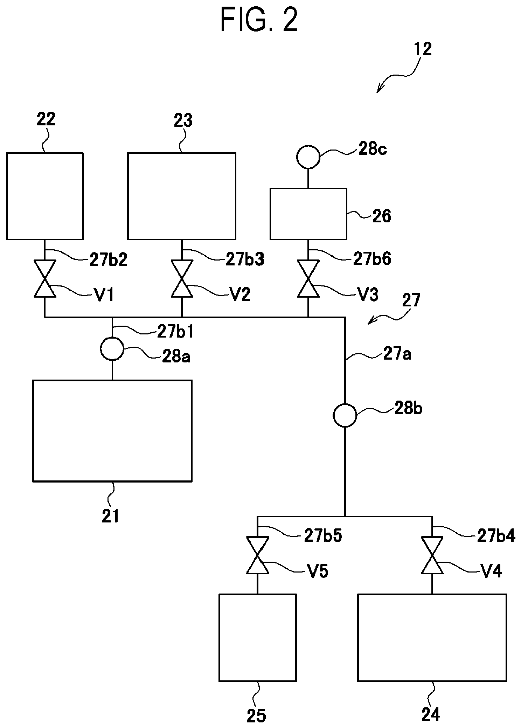

As shown in FIG. 2, the culture vessel 21, a cell bag 22, a supply bag 23, a drainage bag 24, and a recovery bag 25 are disposed in the cultivator 12. The culture vessel 21 is a substantially rectangular parallelepiped container capable of culturing cells in a plurality of culture layers. When performing cell culture, cells are caused to adhere to a bottom surface of each culture layer of the culture vessel 21. As the culture progresses, the cells proliferate along the bottom surface of each culture layer of the culture vessel 21. A cell suspension to be supplied to the culture vessel 21 is accommodated in the cell bag 22. The cell suspension is obtained by suspending cells in a culture medium. A culture medium to be supplied to the culture vessel 21 is accommodated in the supply bag 23. In addition, the culture medium constituting the cell suspension accommodated in the cell bag 22 and the culture medium accommodated in the supply bag 23 are, for example, the same type of culture medium. The drainage bag 24 accommodates ae culture medium discharged from the culture vessel 21 when the culture medium is replaced. The recovery bag 25 is for recovering the culture medium or the cell suspension containing the cells after completion of the culture.

In addition, a sensor 26 for measuring a lactic acid concentration in the culture medium in the culture vessel 21 and a lactic acid concentration in the culture medium in the supply bag 23 is disposed in the cultivator 12. In the present embodiment, the sensor 26 is a sensor that measures a physical quantity by utilizing an enzyme reaction, and is a sensor that measures a lactic acid concentration as the physical quantity. Cells intake glucose contained in the culture medium and discharge lactic acid by metabolism. That is, the lactic acid is a metabolite of the cells that increases with proliferation of the cells. The sensor 26 is, for example, an enzyme sensor manufactured by Sartorius. A configuration of the sensor 26 will be described below. Although not shown, the sensor 26 includes a container capable of accommodating a small amount of culture medium and buffer solution, an electrode immersed in the buffer solution accommodated in the container, an enzyme coated on the electrode, and an electron transfer substance for transferring generated electrons to the electrode. The container has a portion accommodating the culture medium and a portion accommodating the buffer solution, and the respective portions are partitioned by a dialysis membrane. Components contained in the small amount of culture medium, which is sent from the culture vessel 21 or the supply bag 23 to the container of the sensor 26, flow into the buffer solution via the dialysis membrane. The buffer solution into which the components contained in the culture medium have flowed is transferred to the electrode of the sensor 26. The electrode is, for example, a platinum electrode. The enzyme is lactate oxidase. A procedure for the sensor 26 to measure the lactic acid concentration is as follows. First, the lactic acid contained in the culture medium is oxidized by the lactate oxidase to generate pyruvic acid and hydrogen peroxide. Subsequently, by applying a voltage to the electrode, the hydrogen peroxide is decomposed into oxygen and hydrogen ions, and electrons are generated. Subsequently, the generated electrons are measured as a current, and a lactic acid concentration is calculated based on the measured current.

The sensor 26 measures the lactic acid concentration in the culture medium in the culture vessel 21 by the above-mentioned principle utilizing the enzyme reaction. However, due to an unintended reaction of certain interfering substances in the culture medium with the enzyme or the electrode of the sensor 26, the sensor 26 may erroneously recognize an output value derived from the interfering substances as the lactic acid concentration. The "certain interfering substances" refer to one or more chemical substances contained in the culture medium, which may generate electrons by a reaction with the enzyme or the electrode of the sensor 26. The interfering substances in the present embodiment are, for example, substances that may chemically react with the lactate oxidase or the platinum electrode of the sensor 26. Therefore, with respect to the lactic acid concentration in the culture medium in the culture vessel 21 during culturing the cells, a measured value of the lactic acid concentration measured by the sensor 26 includes, in addition to an actual lactic acid concentration, the erroneous output value derived from the interfering substances in the culture medium. Thus, in the present embodiment, the sensor 26 measures the lactic acid concentration in the culture medium in the culture vessel 21 during culturing the cells as a first physical quantity, and measures an output value of a lactic acid concentration in the culture medium in the supply bag 23, which is the culture medium supplied to the culture vessel 21, as an error output value derived from interfering substances. Then, the calculator 14, which will be described later, calculates a corrected physical quantity by subtracting the error output value from the value of the first physical quantity. It can be said that the corrected physical quantity is a true lactic acid concentration that is not affected by the output value derived from the interfering substances in the culture medium in the culture vessel 21. The calculator 14 can accurately calculate the number of cells by calculating the number of cells in the culture vessel 21 based on the corrected physical quantity. In addition, in the present embodiment, the single sensor 26 measures both the first physical quantity, which is the lactic acid concentration in the culture medium in the culture vessel 21, and the error output value, which is the output value of the lactic acid concentration in the culture medium supplied to the culture vessel 21.

Interiors of the culture vessel 21, the cell bag 22, the supply bag 23, the drainage bag 24, the recovery bag 25, and the container of the sensor 26 to which the culture medium is supplied are kept aseptic.

As shown in FIG. 2, the culture vessel 21, the cell bag 22, the supply bag 23, the drainage bag 24, the recovery bag 25, and the sensor 26 are connected by a connection path 27. The culture vessel 21, the cell bag 22, the supply bag 23, the drainage bag 24, and the recovery bag 25 are detachably attached to the connection path 27 while being kept aseptic. The connection path 27 is, for example, a resin tube. The connection path 27 has a main path 27a and a branch path 27b. The branch path 27b is a path branching from the main path 27a, and includes six branch paths 27b1 to 27b6.

The branch path 27b1 is a path that connects the culture vessel 21 and the main path 27a. The branch path 27b2 is a path that connects the cell bag 22 and the main path 27a. The branch path 27b3 is a path that connects the supply bag 23 and the main path 27a. The branch path 27b4 is a path that connects the drainage bag 24 and the main path 27a. The branch path 27b5 is a path that connects the recovery bag 25 and the main path 27a. The branch path 27b6 is a path that connects the sensor 26 and the main path 27a. As shown in FIG. 2, the branch paths 27b1, 27b2, 27b3 and 27b6, and the branch paths 27b4 and 27b5 are connected to opposite sides of the main path 27a with a second pump 28b, which will be described later, interposed therebetween.

A valve V1 is provided in the branch path 27b2. When the valve V1 is open, a liquid can pass through the branch path 27b2, and when the valve V1 is closed, a liquid cannot pass through the branch path 27b2. A valve V2 is provided in the branch path 27b3. When the valve V2 is open, a liquid can pass through the branch path 27b3, and when the valve V2 is closed, a liquid cannot pass through the branch path 27b3. A valve V3 is provided in the branch path 27b6. When the valve V3 is open, a liquid can pass through the branch path 27b6, and when the valve V3 is closed, a liquid cannot pass through the branch path 27b6. A valve V4 is provided in the branch path 27b4. When the valve V4 is open, a liquid can pass through the branch path 27b4, and when the valve V4 is closed, a liquid cannot pass through the branch path 27b4. A valve V5 is provided in the branch path 27b5. When the valve V5 is open, a liquid can pass through the branch path 27b5, and when the valve V5 is closed, a liquid cannot pass through the branch path 27b5. Opening and closing each valve is controlled by the controller 13.

In addition, as shown in FIG. 2, a first pump 28a is provided in the branch path 27b1. The second pump 28b is provided in the main path 27a. A third pump 28c is attached to the sensor 26. Each pump is, for example, a peristaltic pump in which a tube is sandwiched by metal rotors and drawing-in and sending-out of a fluid is performed by rotating the rotors, and which includes a motor configured to drive the rotors. Alternatively, each pump may also be a syringe pump that includes a cylindrical cylinder, a piston configured to reciprocate in the cylinder to perform drawing-in and sending-out of a fluid, and a motor configured to drive the piston.

The controller 13 is connected to the valves V1 to V5, the first pump 28a, the second pump 28b, the third pump 28c, and the like. The controller 13 controls opening and closing of the valves V1 to V5 and driving of each pump. The controller 13 may be connected to each valve and each pump either wirelessly or electrically.

The calculator 14 is connected to the sensor 26 and calculates the number of cells based on the lactic acid concentration in the culture medium measured by the sensor 26. A method of calculating the number of cells by the calculator 14 will be described in detail later.

### (Process of Culturing Cells)

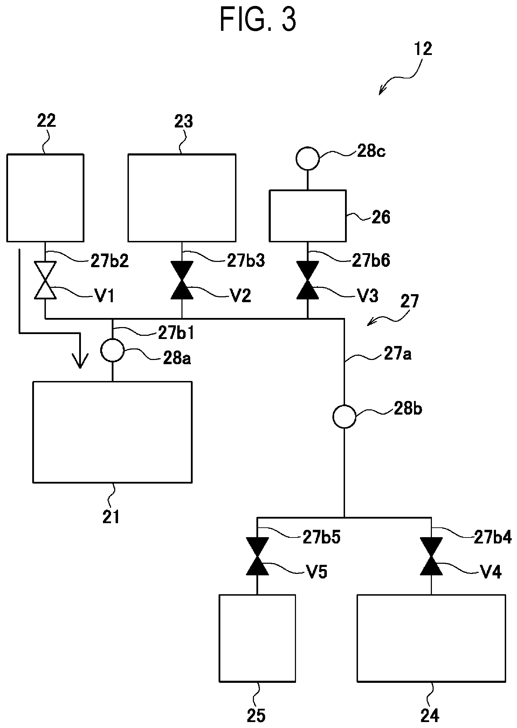

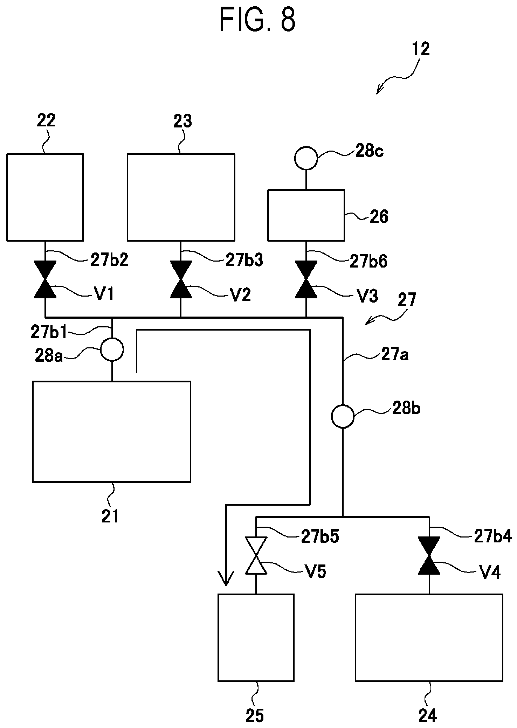

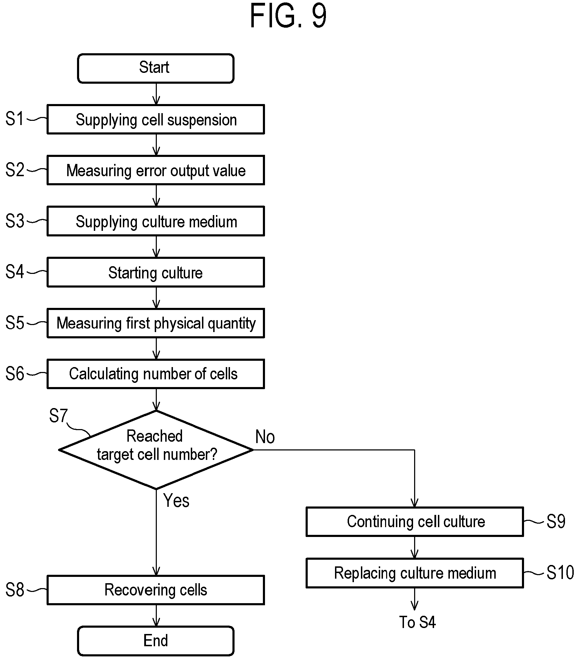

Next, a procedure for culturing cells in the cell culture device 1 according to the present embodiment will be described below with reference to FIGS. 3 to 9. In cell culture, a cell number calculation method is executed to calculate the number of cells in the culture vessel 21. FIG. 9 shows a flowchart when culturing cells.

First, when performing cell culture, the culture vessel 21 in an empty state, the cell bag 22 accommodating a cell suspension containing original cells and a culture medium, the supply bag 23 accommodating a culture medium, the drainage bag 24 in an empty state, and the recovery bag 25 in an empty state are set in advance in the cultivator 12. In other words, the culture vessel 21, the cell bag 22, the supply bag 23, the drainage bag 24, and the recovery bag 25 are connected to the connection path 27 in advance. The connection of each bag to the connection path 27 is performed by an operator. At this time, each of the valves V1 to V5 is in a closed state. In addition, in FIGS. 3 to 8, valves in black indicate a closed state, and valves in white indicate an open state.

Subsequently, as shown in FIG. 3, the controller 13 opens the valve V1 and drives the first pump 28a. Thus, the cell suspension accommodated in the cell bag 22 is sent to the culture vessel 21 (step S1) (*see* the solid arrow in FIG. 3). At this time, an entire amount of the cell suspension accommodated in the cell bag 22 is sent to the culture vessel 21.

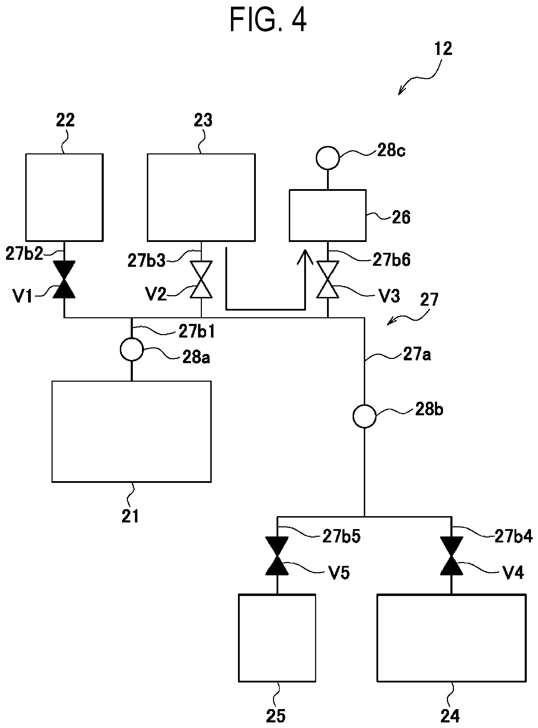

Subsequently, as shown in FIG. 4, the controller 13 closes the valve V1, opens the valves V2 and V3, and drives the third pump 28c. Thus, a part of the culture medium in the supply bag 23 is sent to the sensor 26 (*see* the solid arrow in FIG. 4). At this time, an amount of culture medium sent from the supply bag 23 to the sensor 26 is a part of the culture medium accommodated in the supply bag 23, and is, for example, 40 ml. The sensor 26 measures an output value of a lactic acid concentration derived from interfering substances in the culture medium sent from the supply bag 23 as an error output value (step S2). The output value of the lactic acid concentration referred to herein is a value output as a physical quantity similar to a lactic acid concentration, but is different from an actual lactic acid concentration. A process of measuring the error output value corresponds to a second measurement process according to the present embodiment. The culture medium sent from the supply bag 23 to the sensor 26 is sent to the drainage bag 24 at a predetermined timing after the error output value is measured by the sensor 26 and before step S5, which will be described later, is performed. For example, the culture medium sent from the supply bag 23 to the sensor 26 is sent to the drainage bag 24 after the error output value is measured by the sensor 26 and before step S3, which will be described below, is performed.

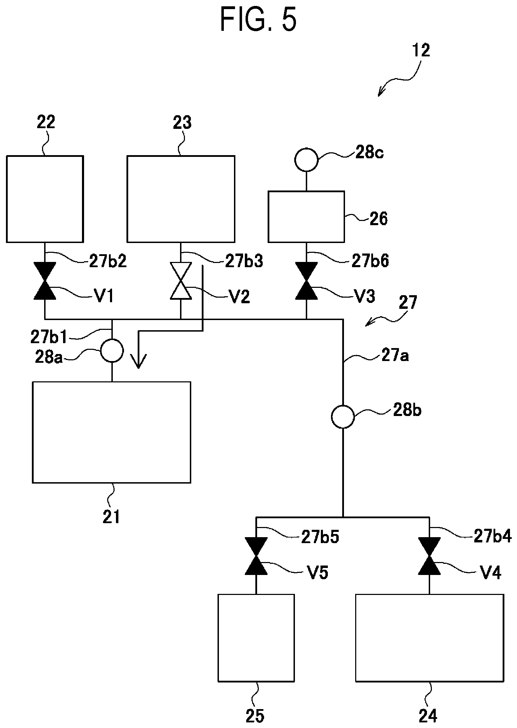

Subsequently, as shown in FIG. 5, the controller 13 closes the valve V3 and drives the first pump 28a. Thus, the culture medium accommodated in the supply bag 23 is sent to the culture vessel 21 (step S3) (*see* the solid arrow in FIG. 5). At this time, a required amount of the culture medium accommodated in the supply bag 23 is sent to the culture vessel 21. The required amount varies according to a type of the culture vessel 21, but is, for example, 200 ml to 300 ml per culture layer of the culture vessel 21. That is, for example, for the culture vessel 21 having five culture layers, the amount of culture medium supplied from the supply bag 23 is 1000 ml to 1500 ml. In the present embodiment, the supply bag 23 accommodates an amount of culture medium, which can be supplied to the culture vessel 21 having, for example, ten culture layers at a time.

In addition, when the amount of culture medium accommodated in the supply bag 23 decreases as the culture medium is supplied from the supply bag 23 to the culture vessel 21, the culture medium stored in the refrigerating storage 11 is newly sent to the supply bag 23. The supply bag 23 and a container (not shown) that accommodates the culture medium in the refrigerating storage 11 are connected by a tube (not shown) or the like. In one culture cycle from starting cell culture to recovering cells, the same lot of culture medium is supplied from the refrigerating storage 11 to the supply bag 23. When the cells are proliferated in one culture cycle and then another culture cycle is performed for sub-culturing the cells, different lots of culture medium may be used in the respective culture cycles.

When the cell suspension is sent from the cell bag 22 to the culture vessel 21 and the culture medium is sent from the supply bag 23 to the culture vessel 21, cell culture is started in the culture vessel 21 (step S4). At this time, the cells in the cell suspension adhere to the bottom surfaces of the multiple culture layers of the culture vessel 21. When the cell culture is being performed, a temperature and a CO₂ concentration in the cultivator 12 are adjusted appropriately. The temperature is adjusted to, for example, 37 degrees C. The CO₂ concentration is adjusted to, for example, 5%. Further, when the cell culture is being performed, each of the valves V1 to V5 is in a closed state.

### (Calculation of Number of Cells)

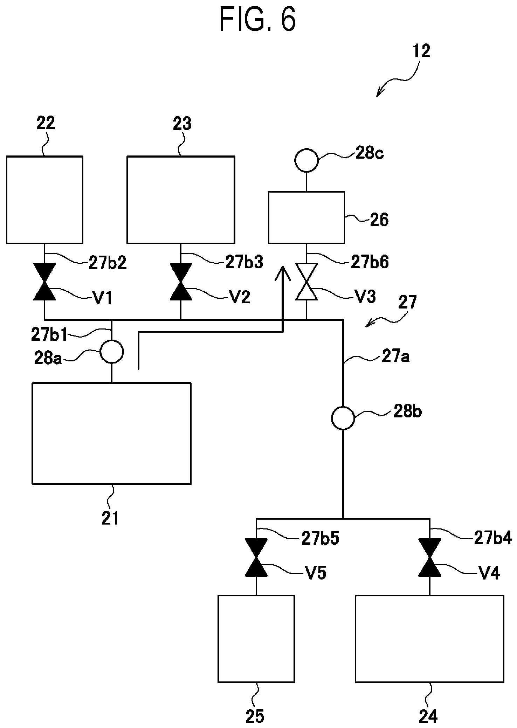

When a predetermined time has elapsed after starting the cell culture, the number of cells in the culture vessel 21 is calculated. A timing for calculating the number of cells in the culture vessel 21 is set in advance, for example. Specifically, first, as shown in FIG. 6, the controller 13 opens the valve V3 and drives the third pump 28c. Thus, a part of the culture medium in the culture vessel 21 is sent to the sensor 26 (*see* the solid arrow in FIG. 6). At this time, an amount of culture medium sent from the culture vessel 21 to the sensor 26 is a part of the culture medium accommodated in the culture vessel 21, and is, for example, 20 ml. In addition, at this time, the cells adhering to the bottom surface of each culture layer of the culture vessel 21 are not stripped. The sensor 26 measures a lactic acid concentration in the culture medium sent from the culture vessel 21 as a first physical quantity (step S5). After the sensor 26 measures the first physical quantity, the controller 13 closes the valve V3. A process of measuring the first physical quantity corresponds to a first measurement process according to the present disclosure.

Subsequently, the calculator 14 calculates the number of cells in the culture vessel 21 based on the first physical quantity and the error output value measured by the sensor 26 (step S6). Specifically, the calculator 14 calculates the number of cells in the culture vessel 21 based on a corrected physical quantity, which is calculated by subtracting the error output value from a value of the first physical quantity. The number of cells corresponding to the corrected physical quantity is predetermined, for example, for each type of the cells or for each type of the culture medium. A process of calculating the number of cells in the culture vessel 21 by the calculator 14 corresponds to a cell number calculation process according to the present disclosure. Subsequently, the controller 13 determines whether or not the number of cells in the culture vessel 21 calculated by the calculator 14 has reached a preset target cell number (step S7).

When the number of cells in the culture vessel 21 has reached the target cell number (S7: "Yes"), recovery of the cells are performed (step S8). A process of recovering the cells will be described later. When the number of cells in the culture vessel 21 has not reached the target cell number (S7: "No"), the cell culture continues in the culture vessel 21 (step S9).

### (Replacement of Culture Medium)

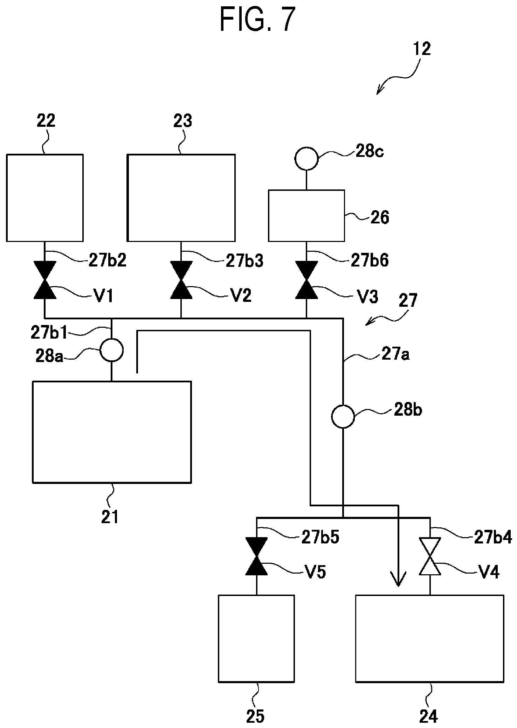

When the cell culture is performed for a predetermined time, replacement of the culture medium in the culture vessel 21 is performed (step S10). The predetermined time is a time duration set in advance according to a type of the cell and a type of the culture medium. To explain the replacement of the culture medium in detail, first, as shown in FIG. 7, the controller 13 opens the valve V4 and drives the second pump 28b. As a result, the culture medium in the culture vessel 21 is sent to the drainage bag 24 (*see* the solid arrow in FIG. 7). At this time, almost the entire amount of the culture medium used for culturing the cells in the culture vessel 21 is sent to the drainage bag 24. Subsequently, the controller 13 closes the valve V4, opens the valve V2, and drives the first pump 28a. As a result, the culture medium accommodated in the supply bag 23 is sent to the culture vessel 21 (*see* the solid arrow in FIG. 5). At this time, a required amount of the culture medium accommodated in the supply bag 23 is sent to the culture vessel 21. The required amount is as described above. That is, the required amount varies according to the type of the culture vessel 21, but is, for example, 200 ml to 300 ml per culture layer of the culture vessel 21. As described above, the replacement of the culture medium in the culture vessel 21 is performed.

When the replacement of the culture medium in the culture vessel 21 is completed, the process returns to step S4, and cell culture is restarted in the culture vessel 21. Then, when a predetermined time has elapsed after the restart of the cell culture, the sensor 26 measures the lactic acid concentration in the culture medium in the culture vessel 21 as the first physical quantity (step S5). Thereafter, the calculator 14 calculates the number of cells in the culture vessel 21 based on the first physical quantity measured again by the sensor 26 and the error output value measured by the sensor 26 as the output value of the lactic acid concentration in the culture medium initially supplied to the culture vessel 21 (step S6).

### (Recovery of Cells)

When the number of cells in the culture vessel 21 has reached the target cell number (S7: "Yes"), recovery of the cells is performed (step S8). Specifically, first, the controller 13 opens the valve V4 and drives the second pump 28b. As a result, an entire amount of the culture medium in the culture vessel 21 is sent to the drainage bag 24 (*see* the solid arrow in FIG. 7). Subsequently, the controller 13 closes the valve V14, and a stripping solution bag (not shown) is connected to the branch path 27b2 of the connection path 27 in place of the cell bag 22. The stripping solution bag accommodates a stripping solution for stripping the cells adhered to the bottom surface of each culture layer of the culture vessel 21 from the bottom surface. The cell bag 22 and the stripping solution are exchanged by, for example, an operator. The cell bag 22 and the stripping solution are exchanged while maintaining aseptic. Subsequently, the controller 13 opens the valve V1 and drives the first pump 28a (*see* FIG. 3 for the open/closed state of each valve). As a result, an entire amount of the stripping solution accommodated in the stripping solution bag (not shown) is sent to the culture vessel 21. Subsequently, the controller 13 closes the valve V1, opens the valve V2, and drives the first pump 28a (*see* FIG. 5 for the open/closed state of each valve). As a result, the culture medium accommodated in the supply bag 23 is sent to the culture vessel 21. In this state, the controller 13 closes the valve V2, stops driving the first pump 28a, and stirs the culture medium in the culture vessel 21. Thus, a cell suspension in which the cells are suspended in the culture medium is accommodated in the culture vessel 21. In this state, the controller 13 opens the valve V5 and drives the second pump 28b. As a result, the cell suspension in the culture vessel 21 is sent to the recovery bag 25. This completes the recovery of the cells.

As described above, the cell culture process in the cell culture device 1 is completed.

### (Effects)

As described above, the cell culture device 1 according to the present embodiment includes the sensor 26 that measures the lactic acid concentration in the culture medium. The sensor 26 measures, as the first physical quantity, the lactic acid concentration in the culture medium in the culture vessel 21 during the cell culture. The sensor 26 also measures, as the error output value, the lactic acid concentration in the culture medium in the supply bag 23, i.e., the output value of the lactic acid concentration in the culture medium supplied to the culture vessel 21. Further, the cell culture device 1 includes the calculator 14 that calculates the number of cells in the culture vessel 21 based on the value of the corrected physical quantity calculated by subtracting the error output value from the value of the first physical quantity. In addition, the cell number calculation method according to the present embodiment executes the first measurement process of measuring the first physical quantity, the second measurement process of measuring the error output value, and the cell number calculation process of calculating the number of cells in the culture vessel 21 based on the corrected physical quantity calculated by subtracting the error output value from the value of the first physical quantity.

According to the present embodiment, the corrected physical quantity is calculated by subtracting the error output value derived from the substance originally contained in the culture medium from the value of the first physical quantity in the culture medium in the culture vessel 21. Thus, it is possible to obtain a concentration of lactic acid, which is a metabolite of cells and is not affected by substances contained in the culture medium, i.e., a corrected physical quantity associated with the number of cells in the culture vessel 21. Therefore, by deriving the number of cells based on the corrected physical quantity, it is possible to accurately calculate the number of cells in the culture vessel 21.

In addition, in the cell culture device 1 according to the present embodiment, the single sensor 26 measures both the first physical quantity and the error output value. Even sensors of the same type may be slightly different in measurement capability due to difference in lots, and the like. Thus, when the lactic acid concentration in the culture medium in the culture vessel 21 and the lactic acid concentration in the culture medium in the supply bag 23 are measured by different sensors, measurement errors may be generated between the sensors. According to the present embodiment, since the lactic acid concentration in the culture medium in the culture vessel 21 and the lactic acid concentration in the culture medium in the supply bag 23 are measured by the single sensor 26, it is possible to eliminate measurement errors between sensors.

In addition, in the present embodiment, the physical quantity of the metabolite of cells that increases with cell proliferation is the lactic acid concentration. In general, a concentration of a substance with a large total amount can be measured more accurately than a concentration of a substance with a small total amount. Since a discharge amount of lactic acid as a metabolite of cells is large, the lactic acid concentration can be measured more accurately than concentrations of other substances.

### (Modifications)

Modifications to the above-described embodiment will be described below. Components having the same configurations as those of the above-described embodiment will be designated by like reference numerals, and descriptions thereof will be omitted as appropriate.

### (First Modification)

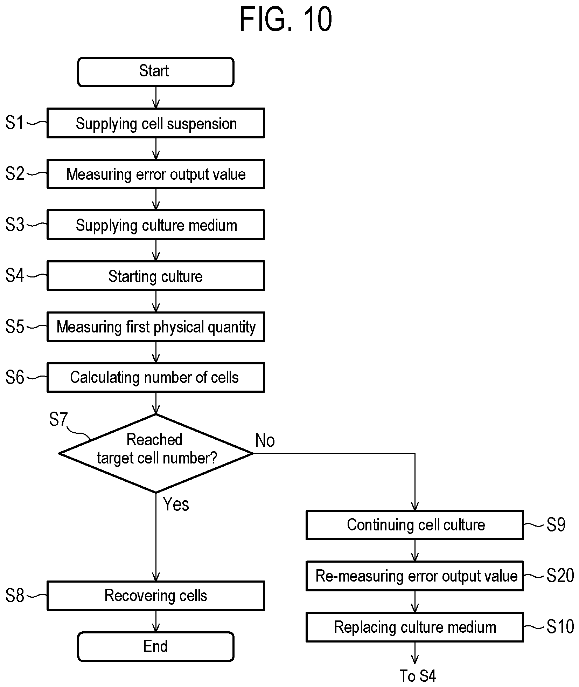

In the above-described embodiment, the sensor 26 first measures the output value of the lactic acid concentration in the culture medium supplied to the culture vessel 21 as the error output value, and does not re-measure the error output value when the culture medium is replaced. However, the sensor 26 may re-measure, as the error output value, the output value of the lactic acid concentration in the culture medium newly supplied to the culture vessel 21 when the replacement of the culture medium is performed. Hereinafter, a description will be given with reference to FIG. 10. In FIG. 10, the same reference numerals are used for steps S1 to S10 common to the above-described embodiment. As shown in FIG. 10, when the culture medium is replaced, the error output value is first re-measured (step S20) before the culture medium is replaced (step S10). Specifically, the controller 13 opens the valves V2 and V3 and drives the third pump 28c. As a result, a part of the culture medium in the supply bag 23 is sent to the sensor 26 (*see* the solid arrow in FIG. 4). At this time, an amount of culture medium sent from the supply bag 23 to the sensor 26 is a part of the culture medium accommodated in the supply bag 23, for example, 20 ml. The sensor 26 measures, as the error output value, the output value of the lactic acid concentration in the culture medium sent from the supply bag 23. That is, the sensor 26 re-measures, as the error output value, the output value of the lactic acid concentration in the culture medium newly supplied to the culture vessel 21 when the replacement of the culture medium is performed. After the sensor 26 measures the error output value, the controller 13 closes the valve V3. After the error output value is re-measured, the culture medium in the culture vessel 21 is replaced (step S10). The procedure for replacing the culture medium is the same as in the above-described embodiment.

That is, in the first modification, the culture medium in the culture vessel 21 is replaced during the culture, and the sensor 26 measures, as the error output value, the output value of the lactic acid concentration in the culture medium newly supplied to the culture vessel 21 when the replacement of the culture medium is performed. In addition, in the cell number calculation method according to the present embodiment, the output value of the lactic acid concentration in the culture medium newly supplied to the culture vessel 21 when the replacement of the culture medium is performed is measured in the second measurement process as the error output value.

The sensor 26 measuring the lactic acid concentration may deteriorate as the measurement is repeated and the measurement capability may be reduced. In such a case, a deviation is generated in the measurement value of the lactic acid concentration according to timings of the measurement by the sensor 26. When a timing of measuring the first physical quantity by the sensor 26 and a timing of measuring the error output value by the sensor 26 are significantly different, the first physical quantity and the error output value are measured by the sensor 26 having variation in measurement capability. The corrected physical quantity calculated from the first physical quantity and the error output value, which are measured by the sensor 26 having variation in measurement capability as described above, may deviate from an actual value. According to the present embodiment, the sensor 26 re-measures, as the error output value, the output value of the lactic acid concentration in the culture medium newly supplied to the culture vessel 21 at the timing of replacing the culture medium. Therefore, it is possible to suppress a significant deviation between the timing of measuring the first physical quantity, which is the lactic acid concentration in the culture medium in the culture vessel 21 during the culture, and the timing of measuring the error output value. Thus, it is possible to suppress a deviation of the corrected physical quantity from the actual value caused by variation in measurement capability of the sensors 26 due to aging degradation. By deriving the number of cells based on the corrected physical quantity obtained as described above, the number of cells in the culture vessel 21 can be calculated accurately.

In addition, when culturing cells, a plurality of culture cycles may be performed. That is, after one culture cycle from starting the cell culture to recovering the cells is completed, a next culture cycle such as sub-culturing the cells may be performed. In this case, a lot for the culture medium used in one culture cycle may be different from a lot for the culture medium used in the next culture cycle. In other words, a lot for the culture medium supplied from the supply bag 23 to the culture vessel 21 in one culture cycle may be different from a lot for the culture medium supplied from the supply bag 23 to the culture vessel 21 in the next culture cycle. Here, the culture media of different lots may be slightly different in composition even when they are the same type. In such a case, for example, the error output value in the culture medium supplied to the culture vessel 21 in the first culture cycle and the error output value in the culture medium supplied to the culture vessel 21 in the next culture cycle will vary due to the difference in the lots for the culture medium. When the variation in the error output value due to the difference in the lots between the culture media is not taken into consideration, the value of the corrected physical quantity calculated by subtracting the error output value from the value of the first physical quantity may deviate from the actual value. In this regard, in the first modification, the sensor 26 re-measures, as an error output value, the output value of the lactic acid concentration in the culture medium newly supplied to the culture vessel 21 when the replacement of the culture medium is performed. Therefore, even when the culture media of different lots are used for a plurality of culture cycles, it is possible to take into account variation in the error output value between culture media of different lots, and to obtain an accurate value of the corrected physical quantity. By deriving the number of cells based on the corrected physical quantity obtained as described above, the number of cells in the culture vessel 21 can be calculated accurately.

### (Other Modifications)

In the above-described embodiment, the single sensor 26 measures both the first physical quantity and the error output value. However, a sensor that measures the first physical quantity and a sensor that measures the error output value may be provided separately.

In the above-described embodiment, the sensor 26 measures the lactic acid concentration as a physical quantity of a metabolite of cells. However, the physical quantity measured by the sensor 26 is not limited to the lactic acid concentration. For example, when cells in a differentiated state are cultured, the physical quantities measured by the sensor 26 may include concentrations of glutamic acid and kynurenine in addition to the lactic acid concentration. Further, when cells in an undifferentiated state are cultured, the physical quantities measured by the sensor 26 may include concentrations of glutamic acid and a 2-aminoadipic acid in addition to the lactic acid concentration.

In the above-described embodiment, the sensor 26 measures the lactic acid concentration in the culture medium in the supply bag 23 when measuring the error output value, which is the lactic acid concentration in the culture medium supplied to the culture vessel 21. However, the sensor 26 may measure the lactic acid concentration in the culture medium in the supply bag 23. For example, the sensor 26 may measure a lactic acid concentration in a culture medium, which is the same type as the culture medium in the supply bag 23 but is different from the culture medium accommodated in the supply bag 23. Specifically, the sensor 26 may measure a lactic acid concentration in a culture medium in a separate supply bag (not shown) from the supply bag 23 connected to the connection path 27 (branch path 27b6). In this case, the culture medium in the supply bag (not shown) having a lactic acid concentration measured by the sensor 26 is then connected to the connection path 27 at a predetermined timing, and is supplied into the culture vessel 21. In addition, the separate supply bag (not shown) is detachably attached to the connection path 27 while maintaining aseptic.

In the above-described embodiment, the calculator 14 that calculates the number of cells in the culture vessel 21 based on the corrected physical quantity is included in the cell culture device 1. However, the calculator 14 may be built in an external PC. In this case, data on the first physical quantity and the error output value measured by the sensor 26 is transmitted to the external PC. In addition, in the cell number calculation method, an operator may calculate the number of cells in the culture vessel 21 corresponding to the corrected physical quantity, based on the corrected physical quantity calculated in the cell culture device 1. Alternatively, the operator may calculate the corrected physical quantity based on the first physical quantity and the error output value, which are measured by the sensor 26, and may further calculate the number of cells in the culture vessel 21 based on the calculated corrected physical quantity.

### EXPLANATION OF REFERENCE NUMERALS

1: cell culture device, 13: controller, 14: calculator (cell number calculation means), 21: culture vessel, 23: supply bag, 26: sensor, 27: connection path

## Claims

1. A cell culture device, comprising:
a culture vessel configured to accommodate cells and a culture medium to culture the cells;
a sensor configured to measure, with respect to a physical quantity of a metabolite of the cells that increases with proliferation of the cells, a first physical quantity, which is the physical quantity in the culture medium in the culture vessel during the culture of the cells, and an error output value, which is an output value of the physical quantity in the culture medium supplied to the culture vessel; and
a cell number calculator configured to calculate a number of cells in the culture vessel based on a corrected physical quantity calculated by subtracting the error output value from a value of the first physical quantity.

2. The cell culture device of Claim 1, wherein the sensor measures both the first physical quantity and the error output value.

3. The cell culture device of Claim 1 or 2, wherein the culture medium in the culture vessel is replaced during the culture, and
wherein the sensor measures, as the error output value, an output value of the physical quantity in the culture medium newly supplied to the culture vessel when the replacement of the culture medium is performed.

4. The cell culture device of any one of Claims 1 to 3, wherein the first physical quantity is a lactic acid concentration.

5. A cell number calculation method of calculating a number of cells in a culture vessel configured to accommodate the cells and a culture medium and culture the cells, comprising:
a first measurement process of measuring, with respect to a physical quantity of a metabolite of the cells that increases with proliferation of the cells, a first physical quantity which is the physical quantity in the culture medium in the culture vessel during the culture of the cells;
a second measurement process of measuring an error output value which is an output value of the physical quantity in the culture medium supplied to the culture vessel; and
a cell number calculation process of calculating the number of cells in the culture vessel based on a corrected physical quantity calculated by subtracting the error output value from a value of the first physical quantity.

6. The cell number calculation method of Claim 5, wherein the culture medium in the culture vessel is replaced during the culture, and
wherein in the second measurement process, an output value of the physical quantity in the culture medium newly supplied to the culture vessel when the replacement of the culture medium is performed is measured as the error output value.
